# EUROPEAN PATENT APPLICATION

(11) **EP 4 775 661 A1**
(43) Date of publication of application: **15.07.2026**
(21) Application number: 24862596.4
(22) Date of filing: 22.08.2024
(51) Int. Cl.: C12M 3/00, C12M 1/00, C12N 1/00, C12N 5/071

(54) **CELL CULTURING DEVICE AND CELL CULTURING METHOD**

(30) Priority: 06.09.2023 JP 2023144723
(71) Applicant: Integriculture Inc., Tokyo 113-0033 (JP); Hamano Products Co., Ltd., Tokyo 131-0041 (JP)
(72) Inventor: KOWAKA Masanobu, Tokyo 113-0033 (JP); NAGASHIMA Hiroaki, Tokyo 131-0041 (JP)
(74) Representative: V.O.
(86) International application number: PCT/JP2024/029793
(87) International publication number: WO 2025/052943

(57) **Abstract**

The present invention prevents a decrease in cell culturing efficiency by preventing detachment of cells adhered to scaffolds. A cell culturing device (100) for culturing cells includes: a culture tank (10) for retaining a culture medium (50); partitioning means (20) for partitioning the culture tank into at least a first space (R1) and a second space (R2); and stirring means (30) for stirring the culture medium. The first space (R1) accommodates scaffolds (40) to which cells adhere. The second space (R2) is where the stirring means (30) are provided. The partitioning means (20) are configured to allow the culture medium to pass therethrough, and to disable entry of the scaffolds to the second space from the first space.

## Description

### Technical Field

The present invention relates to a cell culturing device and a cell culturing method.

### Background Art

In recent years, countries around the world have been developing truly sustainable food production systems to achieve the "Sustainable Development Goals (SDGs)", particularly with developments in "cell-based foods" showing significant expansion. As a background technology in the present technical field, for example, a method for culturing tissue cell cultures in liquid using indirect aeration via a gas-permeable membrane of a tubular shape of a permeator is described in Patent Literature 1. As a device for implementing this method, Patent Literature 1 discloses a configuration in which a tubular permeator forms a vertical central tube inside a container of a cell culturing device. A stirrer is fixed to a lower portion of the permeator, with rotation of the stirrer causing a gas to diffuse into a liquid.

### Citation List

### Patent Literature

Patent Literature 1: Japanese Examined Patent Application Publication No. Hei3(1991)-021149

### Summary of Invention

### Technical Problem

However, an upper portion of the tubular permeator of the cell culturing device described in Patent Literature 1 is open, which may allow a cell culture material to enter the interior from the upper portion of the permeator. When the cell culture material attached to a microcarrier enters the interior of the permeator, the cell culture material is sheared by the stirrer. As a result, cells may detach from the microcarrier or be destroyed, which may lead to a decrease in cell culturing efficiency.

The present invention has been made in view of the above circumstances, and its purpose is to prevent cells that adhere to scaffolds from detaching, thereby suppressing a decrease in cell culturing efficiency.

### Solution to Problem

To achieve the purpose described above, a first aspect of the present invention is a cell culturing device for culturing cells, the cell culturing device including: a culture tank for retaining a culture medium; partitioning means for partitioning the culture tank into at least a first space and a second space; and stirring means for stirring the culture medium, where the first space is a space accommodating scaffolds to which the cells adhere, the second space is a space in which the stirring means are provided, and the partitioning means are configured to allow the culture medium to pass therethrough, and to disable entry of the scaffolds to the second space from the first space.

To achieve the purpose described above, a second aspect of the present invention is a cell culturing method using the cell culturing device described in the first aspect described above, the cell culturing method including: a step of supplying scaffolds to the first space and supplying the culture medium to the culture tank; a step of driving the stirring means to stir the culture medium; a step of removing a portion of the scaffolds from the first space; and a step of supplying new scaffolds to the first space and mixing them with the scaffolds remaining in the first space.

### Advantageous Effects of Invention

According to the present invention, the cells that adhere to the scaffolds can be prevented from detaching, thereby suppressing a decrease in cell culturing efficiency. Note that problems, configurations and effects other than those described above will be made clear through the description of the following embodiments.

### Brief Description of Drawings

FIG. 1 is a diagram schematically illustrating an overall configuration of a cell culturing device and a longitudinal cross-section of a culture tank according to a first embodiment of the present invention.
FIG. 2 is a diagram schematically illustrating a transverse cross-section of the culture tank.
FIG. 3 is a perspective view of a cylindrical member provided inside the culture tank.
FIG. 4 is a flowchart illustrating a procedure for cell culturing.
FIG. 5 is a flowchart illustrating a procedure for a collection and replenishment process of scaffolds.
FIG. 6 is a diagram schematically illustrating the overall configuration of the cell culturing device and the longitudinal cross-section of the culture tank according to a second embodiment of the present invention.
FIG. 7 is a perspective view of the cylindrical member provided inside the culture tank.
FIG. 8A is a diagram illustrating a state in which the diameter of the cylindrical member is increased.
FIG. 8B is a diagram illustrating a state in which the diameter of the cylindrical member is decreased.
FIG. 9 is a flowchart illustrating the procedure for the collection and replenishment process of scaffolds according to the second embodiment.
FIG. 10A is a diagram schematically illustrating the longitudinal cross-section of the cell culturing device according to a first modification.
FIG. 10B is a diagram schematically illustrating the transverse cross-section of the culture tank shown in FIG. 10A.
FIG. 11A is a diagram schematically illustrating the transverse cross-section of the cell culturing device according to a second modification.
FIG. 11B is an enlarged view of portion B shown in FIG. 11A.
FIG. 12A is a diagram illustrating a variation in the shape of the culture tank.
FIG. 12B is a diagram illustrating a variation in the shape of the culture tank.
FIG. 12C is a diagram illustrating a variation in the shape of the culture tank.
FIG. 12D is a diagram illustrating a variation in the shape of the culture tank.
FIG. 13 is a diagram schematically illustrating the longitudinal cross-section of the cell culturing device according to a fourth modification.
FIG. 14 is a diagram schematically illustrating the longitudinal cross-section of the cell culturing device according to a fifth modification.
FIG. 15A is a plan view of a basket.
FIG. 15B is a longitudinal cross-sectional view of the basket.
FIG. 16A is a plan view of the basket according to a sixth modification.
FIG. 16B is a cross-sectional view along line XVIB-XVIB in FIG. 16A.

### Description of Embodiments

The following describes embodiments of the present invention with reference to the drawings using examples of culturing animal cells.

### First Embodiment

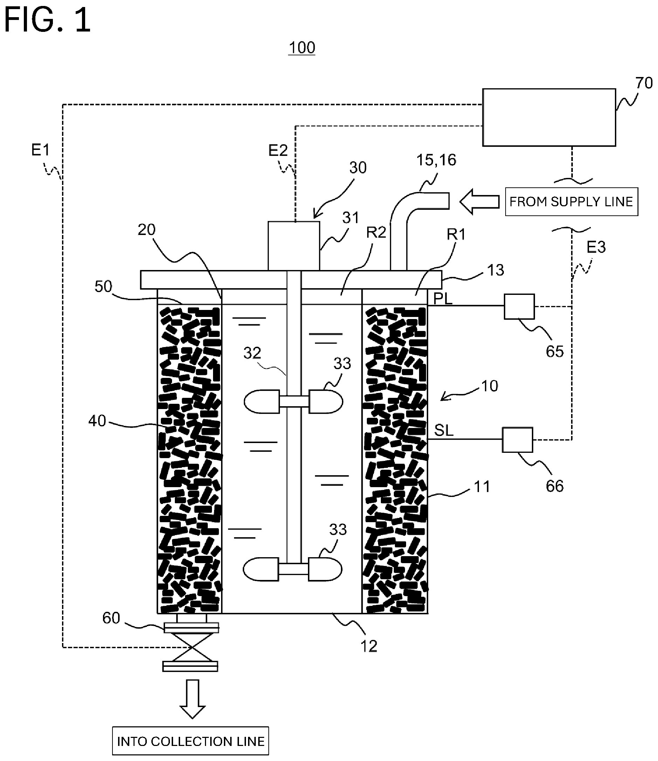

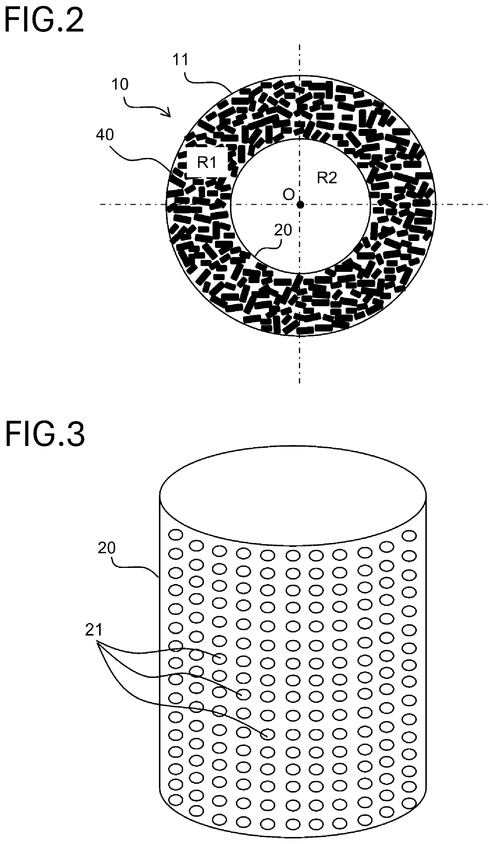

FIG. 1 is a diagram schematically illustrating the overall configuration of a cell culturing device 100 and a longitudinal cross-section of a culture tank 10 according to a first embodiment of the present invention, FIG. 2 is a diagram schematically illustrating a transverse cross-section of the culture tank 10, and FIG. 3 is a perspective view of a cylindrical member 20 provided inside the culture tank 10. Note that in FIG. 2, the illustration of stirring means 30 shown in FIG. 1 is omitted.

As shown in FIG. 1, the cell culturing device 100 according to the present embodiment mainly includes the culture tank 10, the cylindrical member 20 and the stirring means 30.

The culture tank 10 includes a container having a cylindrical shape with a bottom formed by a body portion 11 and a bottom portion 12, and a lid body 13 having a disc shape that covers the upper portion of the body portion 11. The culture tank 10 retains a culture medium 50 therein. Although the culture tank 10 is made of, for example, stainless steel, it may be made of other materials such as glass or silicone resin. Note that, in a case in which the culture tank 10 is made of silicone resin, the culture tank 10 may also be referred to as a culture bag.

The lid body 13 is tightly fitted to the body portion 11 via a gasket, which is not shown in the figure. This seals the interior of the culture tank 10. The lid body 13 is provided with supply tubes 15 and 16. Scaffolds 40 (described later) and the culture medium 50 to be supplied from a supply line are introduced into the culture tank 10 via the supply tubes 15 and 16. The lid body 13 is also provided with various tubes, hoses, nozzles, and the like, which are not shown in the figure, in addition to the supply tubes 15 and 16. These are used for a variety of purposes, including connecting various sensors, supplying oxygen or the like, and sampling.

One solenoid valve 60 is provided on the bottom portion 12 of the culture tank 10. Needless to say, a plurality of solenoid valves 60 may be provided. The body portion 11 of the culture tank 10 is provided with level switches 65 and 66 for detecting the liquid level of the culture medium 50. The level switch 65 detects whether the liquid level of the culture medium 50 is at the proper level PL. The level switch 65 outputs a high-level signal when the liquid level of the culture medium 50 exceeds the proper level PL. Here, the proper level PL refers to a position where the culture medium 50 inside the culture tank 10 is approximately at its full capacity.

In contrast, the level switch 66 detects whether the liquid level of the culture medium 50 is at a specific level SL. The level switch 66 outputs a low-level signal when the liquid level of the culture medium 50 falls below the specific level SL. Here, although in the present embodiment, a level position approximately half that of the proper level PL is defined as the specific level SL, the specific level SL may be arbitrarily set at a position suitable for collection and replenishment of the scaffolds 40.

The solenoid valve 60 is connected to a controller 70 via an electrical wiring E1, and the level switches 65 and 66 are connected to the controller 70 via an electrical wiring E3. The controller 70 controls opening and closing operation of the solenoid valve 60 based on input from the level switches 65 and 66 (the low-level signal or the high-level signal). An arbitrary volume of the scaffolds 40 and the culture medium 50 inside the culture tank 10 is collected (discharged) into a collection line upon opening the solenoid valve 60, and the remaining scaffolds 40 and the culture medium 50 are retained in the culture tank 10 upon closing the solenoid valve 60, after which the scaffolds 40 and the culture medium 50 are further replenished, thereby allowing the animal cells to adhere to the scaffolds 40 replenished.

The cylindrical member (partitioning means) 20 is composed of a member having a cylindrical shape made of, for example, stainless steel. As shown in FIG. 2, the cylindrical member 20 is provided coaxially with the culture tank 10 (on a central axis O) to divide the internal space of the culture tank 10 into two portions concentrically. Specifically, the culture tank 10 is partitioned into a second space R2, which is the internal space of the cylindrical member 20, and a first space R1, which is a space having an annular shape formed between the culture tank 10 and the cylindrical member 20. The first space R1 accommodates the scaffolds 40. The second space R2 is provided with the stirring means 30, while the scaffolds 40 do not exist therein (see FIG. 1).

As shown in FIG. 3, the cylindrical member 20 is provided with numerous holes 21 around its entire circumference. The diameter of the holes 21 is determined to be small enough to prevent the scaffolds 40 from passing therethrough. For example, the diameter of the holes 21 is approximately 500 micrometers. By making the holes 21 of approximately this size, fluid communication between the first space R1 and the second space R2 is established through the numerous holes 21, allowing the culture medium 50 to flow between the first space R1 and the second space R2 via the holes 21 of the cylindrical member 20. In contrast, since the scaffolds 40 are larger than the diameter of the holes 21, the scaffolds 40 cannot enter the second space R2 from the first space R1. In other words, the cylindrical member 20 is configured to allow the culture medium 50 to pass therethrough but prevent the scaffolds 40 from passing therethrough.

Note that, as shown in FIG. 1, the cylindrical member 20 is configured to have approximately the same height as the body portion 11 of the culture tank 10, thus preventing the scaffolds 40 from flowing around the cylindrical member 20 to enter its interior. In this way, the cylindrical member 20 completely separates the culture tank 10 into two spaces, R1 and R2, thereby completely preventing the scaffolds 40 from entering or flowing around the second space R2 from the first space R1.

Here, the cylindrical member 20 is only required to partition the two spaces R1 and R2 so that the scaffolds 40 accommodated in the first space R1 cannot enter the second space R2, without necessarily completely separating the culture tank 10 into two spaces. Even if there is a gap between the cylindrical member 20 and the lid body 13, such a cylindrical member 20 is considered partitioning means according to the present invention, as long as the scaffolds 40 do not enter the second space R2 through the gap.

Note that the cylindrical member 20 may be configured with a wire net having, for example, a mesh size of 14 to 100 (14 to 100 mesh openings per inch). Even this configuration can provide effects equivalent to those of the cylindrical member 20 shown in FIG. 3.

Next, the scaffolds 40 are described. The scaffolds 40 serve as a base to which animal cells adhere, composed of a sheet or three-dimensional structure made of a porous or gel-like material. Materials for the scaffolds 40 are not specifically limited but include resin-based materials such as PET and PP, as well as plant-derived and animal-derived materials. In the present embodiment, the scaffolds 40 are plant-derived or animal-derived edible scaffolds. The scaffolds 40 include, for example, a protein and a polysaccharide. Here, "edible" refers to a composition that can be safely ingested into a human body. Using the scaffolds 40 as a substrate enables the animal cells to adhere and proliferate, thereby producing cell-based foods. Animal cells that adhere to the scaffolds 40 include muscle-derived cells, skin-derived cells, intestine-derived cells, heart-derived cells, brain-derived cells, stomach-derived cells, embryonic membrane-derived cells, liver-derived cells, kidney-derived cells and lung-derived cells. Note that, as shown in FIGS. 1 and 2, the scaffolds 40 are uniformly packed at a high density inside the first space R1, thereby suppressing their flow inside the first space R1.

The stirring means 30 are for stirring the culture medium 50 and, as shown in FIG. 1, includes a motor 31 that serves as a drive source, a rotation shaft 32 and stirring blades 33. Specifically, in the present embodiment, the stirring means 30 of a propeller-type are employed. Arranging the stirring blades 33 in multiple tiers in the height direction allows the culture medium 50 to be distributed more uniformly from the center of the culture tank 10 to the first space R1 throughout the height direction. This further allows the configuration to be applied to a large-scale culture tank 10. Although the stirring blades 33 are arranged in multiple tiers in the present embodiment, the stirring blades 33 may be a single stirring blade extending continuously in the height direction, or a device such as a centrifugal pump that conveys a liquid toward an outer peripheral direction may be employed.

The motor 31 is fixed to the lid body 13. An output shaft and the rotation shaft 32 of the motor 31 are connected via a coupling, which is not shown in the figure. The rotation shaft 32 is provided with a plurality of (for example, three) stirring blades 33 arranged in two tiers, one above the other. When the motor 31 rotates, the rotation shaft 32 rotates in response to a rotation of the motor 31, causing a pair of upper and lower stirring blades 33 to rotate horizontally around the rotation shaft 32. In this way, the culture medium 50 inside the second space R2 is stirred. Then, the culture medium 50 is uniformly introduced into the first space R1 through the numerous holes 21 provided in the cylindrical member 20. Note that the motor 31 is connected to the controller 70 via an electrical wiring E2, allowing its rotation to be controlled in accordance with a command from the controller 70.

The controller 70 is configured to include an arithmetic processing device having a CPU, a ROM and a RAM that serve as memory devices, other peripheral circuits, and the like. The ROM stores a control program, and the CPU reads the control program to execute various processes. Various sensors, which are not shown in the figure, mounted on the cell culturing device 100 are electrically connected to the controller 70. The controller 70 controls, for example, a rotational speed of the motor 31 based on sensor data input and controls the opening and closing operation of the solenoid valve 60 as described later.

### (Cell culturing method)

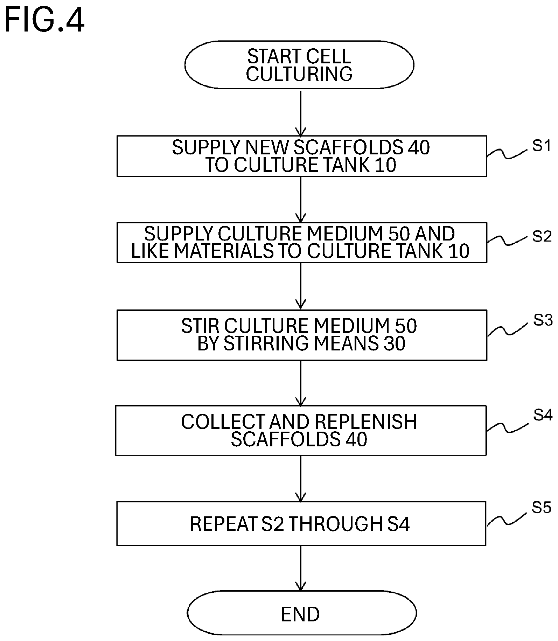

Next, the cell culturing method using the cell culturing device 100 is described. FIG. 4 is a flowchart illustrating a procedure for cell culturing. Each of the processes shown in FIG. 4 is controlled by the controller 70. First, an operator performs a sterilization process inside the cell culturing device 100 as a preliminary step to remove microorganisms and prevent contamination from occurring. Next, in accordance with the command from the controller 70, the scaffolds 40 are supplied to the first space R1 via the supply tubes 15 and 16 from the supply line (S1). Next, the culture medium 50 is supplied to the second space R2 of the culture tank 10 until the liquid surface of the culture medium 50 reaches the proper level PL (S2). At this time, nutrients such as oxygen are also supplied to the culture tank 10 as appropriate. Then, in accordance with the program stored in advance in the controller 70, the controller 70 arbitrarily drives the motor 31 of the stirring means 30 periodically or continuously (S3). In this way, the culture medium 50 is stirred, promoting culture of the animal cells. In other words, the animal cells efficiently proliferate using the scaffolds 40 as the substrate. After the animal cells adhere sufficiently to the scaffolds 40, a portion of the scaffolds 40 is collected, and new scaffolds 40 are replenished (S4). These steps S1 through S4 are repeated until a desired volume of the scaffolds 40 is collected (S5).

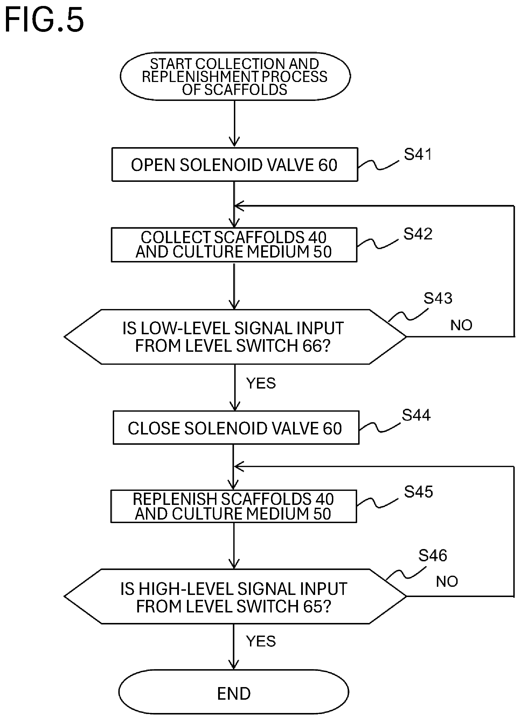

Next, a processing procedure for the collection and replenishment of the scaffolds 40 shown in step S4 is described in detail. FIG. 5 is a flowchart illustrating the procedure for the collection and replenishment process of scaffolds. As shown in FIG. 5, the solenoid valve 60 opens in accordance with the command from the controller 70 (S41), and the scaffolds 40 are discharged into the collection line together with the culture medium 50 (S42). Specifically, a portion of the scaffolds 40 is collected together with the culture medium 50 from the first space R1 of the culture tank 10, causing the liquid surface of the culture medium 50 to gradually decrease.

Next, the controller 70 closes the solenoid valve 60 (S44) based on the low-level signal input from the level switch 66 (S43/YES). Then, the new scaffolds 40 are replenished to the first space R1 via the supply tubes 15 and 16 from the supply line in accordance with the command from the controller 70, and the culture medium 50 is replenished to the second space R2 (S45). Specifically, the same volume of the new scaffolds 40 as the scaffolds 40 collected in S42 is replenished to the first space R1. Therefore, in this S45, old scaffolds 40 (the scaffolds 40 to which cells adhere) and the new scaffolds 40 (the scaffolds 40 to which no cells adhere) are mixed together in the first space R1 at a ratio of approximately 1:1.

Then, as the liquid level of the culture medium 50 rises in response to supply of the culture medium 50, and when the liquid level of the culture medium 50 reaches the proper level PL, the high-level signal is input to the controller 70 from the level switch 65. The controller 70 stops supplying the culture medium 50 based on the high-level signal to terminate the process (S46/YES). In this way, approximately half of the scaffolds 40 to which the animal cells adhere are replaced with the new scaffolds 40.

According to the present embodiment described above, the following effects can be achieved.

The first space R1 and the second space R2 are separated by the cylindrical member 20, and the holes 21 of the cylindrical member 20 are configured to prevent the scaffolds 40 from passing therethrough. Therefore, the scaffolds 40 cannot enter the second space R2. As a result, a collision between the scaffolds 40 and the stirring blades 33 can be prevented, thereby preventing the animal cells that adhere to the scaffolds 40 from detaching. Consequently, according to the present embodiment, cell culturing efficiency can be increased. In addition, the present embodiment also contributes to the achievement of the Sustainable Development Goals (SDGs).

The culture medium 50 is stirred by the stirring blades 33 and distributed uniformly over the scaffolds 40, enabling the animal cells to adhere uniformly to the scaffolds 40. Furthermore, the numerous holes 21 dampen vortices generated during stirring, significantly reducing stress on the animal cells. Therefore, good-quality cell-based foods can be obtained.

Including a process of replacing only about half of the total volume of the scaffolds 40 (S4) also increases cell culturing efficiency compared to the case in which all of the scaffolds 40 are replaced. More specifically, harvesting all of the scaffolds 40 to which the animal cells adhere causes a state in which the number of cells has massively increased to be reset, requiring the cells to be grown again from an initial state in which the number of cells is low when starting the next production process.

The number of cells approximately doubles in one day, thus, if culturing is started with 1 kg of cells as an initial mass of cells, the mass of cells reaches 100 kg after a seven-day culturing period, which means a production mass per day of approximately 14 kg/day. In contrast, if the production is started with 50 kg of cells as the initial mass of cells, since the mass of cells approximately doubles in one day, the mass of cells reaches 100 kg after a one-day culturing period, which means a production mass per day of approximately 50 kg/day.

In other words, retaining roughly half of the old scaffolds 40 while mixing them with the new scaffolds 40 allows the culturing to be started with a larger initial mass of cells by using remaining cells instead of using the cells that are otherwise required to be supplied when starting the next production process. This enables the production mass per day to be increased.

Note that the replacement ratio of the old scaffolds 40 to the new scaffolds 40 can be changed by changing the position of the level switch 66.

Furthermore, the cell culturing device 100 according to the present embodiment has a simple configuration including only the culture tank 10 for retaining the culture medium 50, the cylindrical member 20 provided inside the culture tank 10, and the stirring means 30 for stirring the culture medium 50, thus also offering the advantage of enabling manufacturing equipment to be realized at low cost.

### Second Embodiment

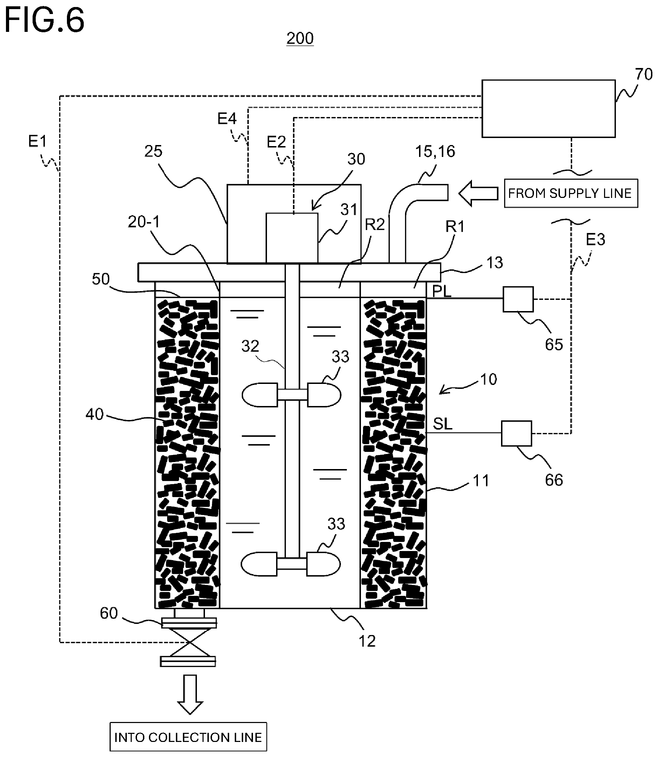

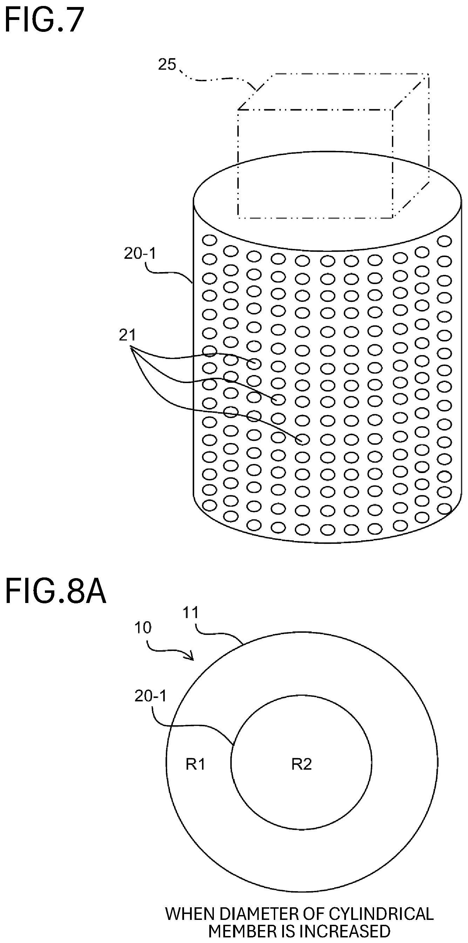

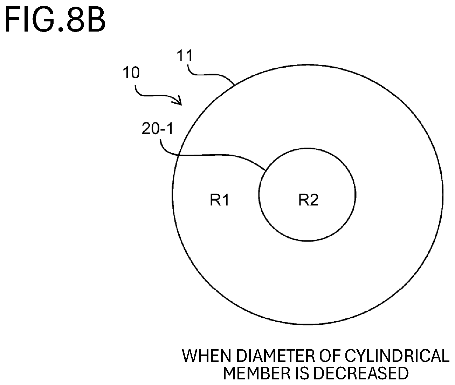

Next, the cell culturing device according to the second embodiment of the present invention is described. FIG. 6 is a diagram schematically illustrating the overall configuration of the cell culturing device 200 and the longitudinal cross-section of the culture tank 10 according to the second embodiment of the present invention, FIG. 7 is a perspective view of the cylindrical member 20-1 provided inside the culture tank 10, and FIGS. 8A and 8B are diagrams illustrating a displacement state of the cylindrical member 20-1. FIG. 8A illustrates a state in which the diameter of the cylindrical member 20-1 is increased, and FIG. 8B illustrates a state in which the diameter of the cylindrical member 20-1 is decreased. Note that in FIGS. 8A and 8B, the illustration of the scaffolds 40 is omitted.

The cell culturing device 200 according to the second embodiment features a configuration in which the diameter of the cylindrical member 20-1 increases and decreases. As shown in FIGS. 6 and 7, expansion and contraction means 25 are provided on an upper end side of the cylindrical member 20-1. The expansion and contraction means 25 are connected to the controller 70 via an electrical wiring E4. The expansion and contraction means 25 increase or decrease the outer diameter of the cylindrical member 20-1 by expanding or contracting the cylindrical member 20-1 in an axial direction in accordance with a command from the controller 70. Note that the cylindrical member 20-1 may adopt any configuration capable of increasing or decreasing its diameter in cooperation with the expansion and contraction means 25, and examples include a stent type, a hinge type, a screw type and a pneumatic expansion type.

The volume ratio of the first space R1 to the second space R2 changes between the state in which the diameter of the cylindrical member 20-1 is increased as shown in FIG. 8A and the state in which the diameter of the cylindrical member 20-1 is decreased as shown in FIG. 8B. Specifically, the volume ratio of the first space R1 is larger in the state in which the diameter of the cylindrical member 20-1 is decreased than in the state in which the diameter of the cylindrical member 20-1 is increased. In other words, when the diameter of the cylindrical member 20-1 is decreased, the space for accommodating the scaffolds 40 expands. Note that in the present embodiment, the diameter of the cylindrical member 20-1 is increased in a normal state, and as described later, the diameter of the cylindrical member 20-1 is decreased when the scaffolds 40 are collected and replenished.

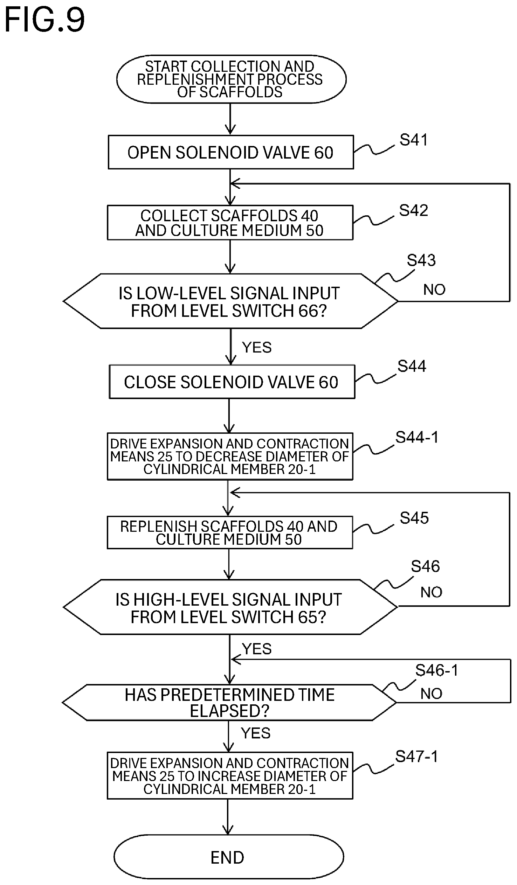

Next, the processing procedure for the collection and replenishment of the scaffolds 40 in the second embodiment is described in detail. FIG. 9 is a flowchart illustrating the procedure for the collection and replenishment process of the scaffolds according to the second embodiment. Processes characteristic of the second embodiment are S44-1, S46-1 and S47-1. Thus, these processes are described in detail, with descriptions of processes that are the same as in the first embodiment omitted.

As shown in FIG. 9, when the solenoid valve 60 closes (S44), the controller 70 drives the expansion and contraction means 25 to decrease the diameter of the cylindrical member 20-1, as shown in FIG. 8B (S44-1). Decreasing the diameter of the cylindrical member 20-1 increases the volume of the first space R1. Then, when the new scaffolds 40 are replenished and the liquid level of the culture medium 50 rises to the proper level PL, the controller 70 maintains this state for a predetermined time. In this state, since the volume of the first space R1 is increased, the scaffolds 40 can move relatively freely inside the first space R1 compared to the state in which the diameter of the cylindrical member 20-1 is increased. Therefore, the culture medium 50 fills the first space R1 more uniformly compared to the first embodiment. Then, when the predetermined time has elapsed (S46-1/YES), the controller 70 drives the expansion and contraction means 25 to increase the diameter of the cylindrical member 20-1 (S47-1).

As described above, according to the second embodiment, similar effects to those of the first embodiment can be achieved. Furthermore, the second embodiment employs a configuration in which the diameter of the cylindrical member 20-1 increases or decreases, allowing the volume of the first space R1 to increase when the scaffolds 40 are replenished to uniformly supply the culture medium 50 to all of the scaffolds 40. In addition, stirring the culture medium 50 using the stirring means 30 while the first space R1 is expanding enables the scaffolds 40 to which the cells adhere and the scaffolds 40 newly replenished to be mixed more uniformly. As a result, the probability of cells cultured on the old scaffolds 40 as the substrate moving to and adhering to the new scaffolds 40 increases, and the number of cells proliferating on the new scaffolds 40 as the substrate increases, leading to a further increase in cell culturing efficiency.

Note that the present invention is not limited to the embodiments described above, various modifications may be made without departing from the spirit of the present invention, and all technical matters included in the technical ideas described in the claims are within the scope of the present invention. While the embodiments described above provide suitable examples, those skilled in the art can realize various alternatives, adjusted examples, modifications or improvements based on the information disclosed in the present description, which are included within the technical scope described in the claims attached hereto.

For example, the embodiments described above can be modified as follows.

### (First Modification)

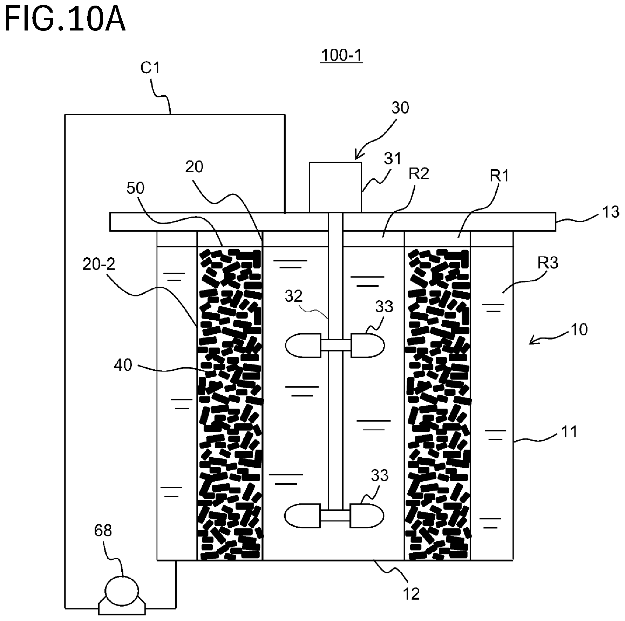

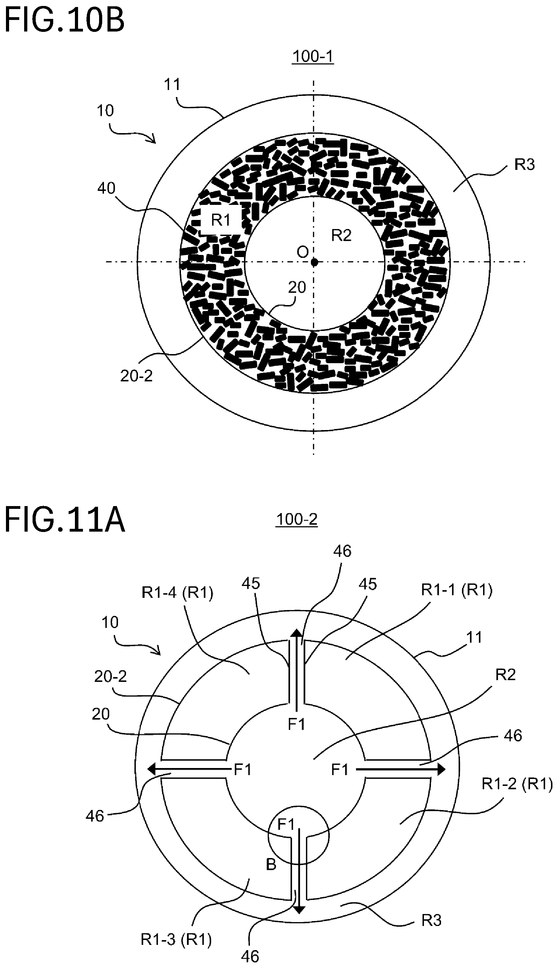

FIG. 10A is a diagram schematically illustrating the longitudinal cross-section of the cell culturing device 100-1 according to a first modification. FIG. 10B is a diagram schematically illustrating the transverse cross-section of the culture tank 10 shown in FIG. 10A. As shown in FIGS. 10A and 10B, the cell culturing device 100-1 according to the first modification has a configuration in which the culture tank 10 is partitioned into three spaces concentrically using two cylindrical members 20 and 20-2. Note that the cylindrical member 20-2 (a second cylindrical member) has the same configuration as the cylindrical member 20 except for its diameter. A third space R3 formed on an outer periphery side of the culture tank 10 (in other words, the third space R3 formed outside of the first space R1 in a radial direction) retains only the culture medium 50, similarly to the second space R2, such that no scaffolds 40 enter (or are present) therein.

In the first modification, a circulation flow passage C1 in which the culture medium 50 circulates between the third space R3 and the second space R2 is provided. The circulation flow passage C1 is provided with a circulation pump 68, and when the circulation pump 68 is driven, the culture medium 50 inside the third space R3 is drawn in by the circulation pump 68 and discharged (returned) to the second space R2 via the circulation flow passage C1. The circulation flow passage C1 and the circulation pump 68 correspond to culture medium circulation means according to the present invention.

When the culture medium 50 inside the third space R3 is drawn in by the circulation pump 68, liquid pressure in the third space R3 slightly drops, causing the liquid pressure in the second space R2 to be relatively higher. This causes the culture medium 50 to flow from the second space R2 to the third space R3 via respective holes 21 of the cylindrical members 20 and 20-2, enabling the culture medium 50 to efficiently circulate.

This further improves the uniformity of the supply of the culture medium 50 to the scaffolds 40, thereby further increasing cell culturing efficiency.

Note that although the first modification has a configuration in which the culture medium 50 is drawn in from the third space R3 and returned to the second space R2, the configuration may be such that the culture medium 50 is drawn in from the second space R2 and returned to the third space R3. Even with such a configuration, circulation of the culture medium 50 enables the culture medium 50 to be supplied uniformly to the scaffolds 40 inside the first space

R1.

### (Second Modification)

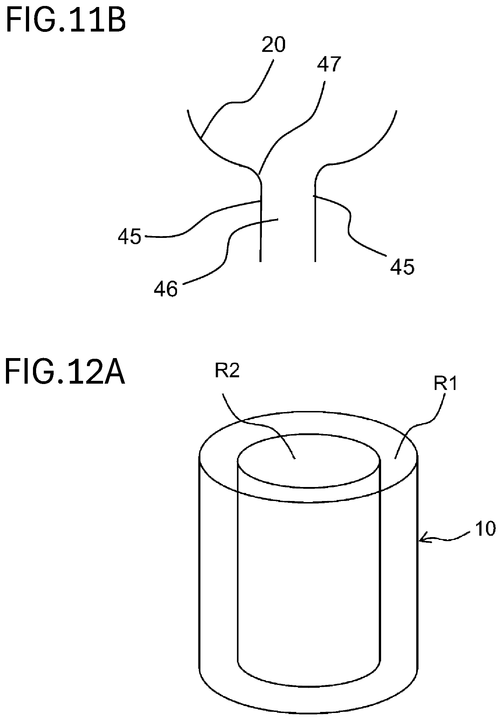

FIG. 11A is a diagram schematically illustrating the transverse cross-section of the cell culturing device 100-2 according to a second modification, and FIG. 11B is an enlarged view of portion B shown in FIG. 11A. As shown in FIG. 11A, the cell culturing device 100-2 according to the second modification also has a configuration, similarly to the first modification, in which the culture tank 10 is partitioned into three spaces concentrically using the two cylindrical members 20 and 20-2. The first space R1 is further partitioned in a circumferential direction into four spaces with approximately equal volumes by a plurality of straightening plates 45, thereby being divided into four spaces: a first divided space R1-1, a second divided space R1-2, a third divided space R1-3 and a fourth divided space R1-4.

The second space R2 and the third space R3 are in fluid communication via a flow passage 46 formed between a pair of straightening plates 45. In the second modification, as shown in FIG. 11A, four pairs of straightening plates 45 are provided at 90-degree intervals in the circumferential direction, with the flow passages 46 each formed between each pair of straightening plates 45. Note that, although not shown in the figure, the straightening plate 45 is provided with numerous holes or slits. Needless to say, the number and shape of the holes or slits are arbitrary.

Next, the effects of the second modification are described. When the culture medium 50 flows from the second space R2 to the first space R1 as described above, turbulence, which is not shown in the figure, may occur in such a case in which the scaffolds 40 having non-uniform shapes are used. The turbulence is difficult to control and may reduce molecular uniformity of nutrients, oxygen, and the like inside the culture tank 10, potentially adversely affecting culturing efficiency. Therefore, as shown in the second modification, forming a flow passage 46 connecting the second space R2 and the third space R3 between a pair of straightening plates 45 enables a portion where the flow is always a laminar flow F1 to be established. In this way, providing a flow passage 46 to establish the laminar flow F1 where the flow of the culture medium 50 can be controlled reduces the occurrence of turbulence in the first space R1, thereby increasing the molecular uniformity inside the culture tank 10 to increase culturing efficiency.

Note that the number of straightening plates 45, in other words, the number of flow passages 46, may be arbitrary. The optimal number of flow passages 46 may be provided depending on conditions such as a size of the culture tank 10 and the shape of the scaffolds 40.

Furthermore, as shown in FIG. 11B, providing an R-shaped portion 47 at the inlet of the flow passage 46 reduces the inlet loss (resistance) of the flow passage 46, thereby enabling a smooth flow of the culture medium 50 from the second space R2 to the third space R3 to maintain a suitable culturing environment.

### (Third Modification)

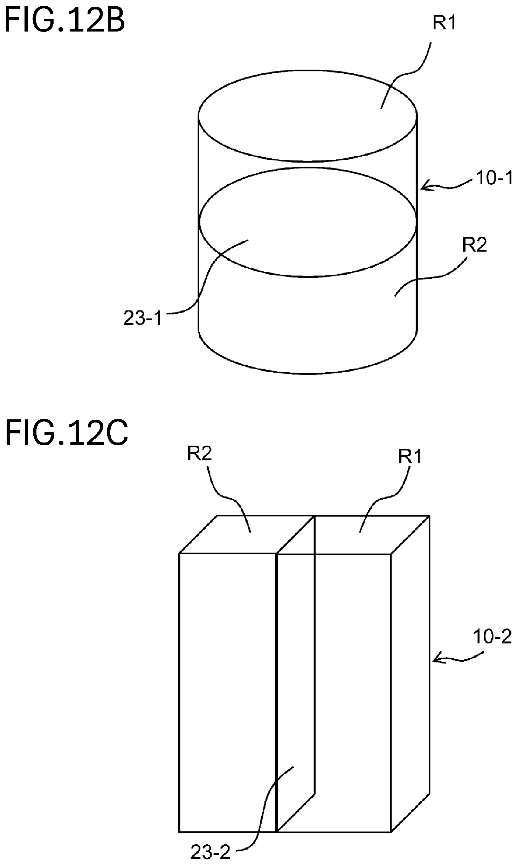

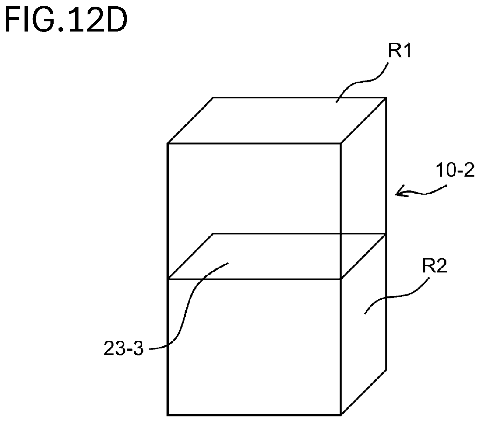

FIGS. 12A through D illustrate variations in the shape of the culture tank 10. FIG. 12A shows a configuration example in which the internal space of the culture tank 10 having a cylindrical shape is partitioned concentrically, corresponding to the first embodiment. FIG. 12B shows a configuration example in which the internal space of the culture tank 10-1 having a cylindrical shape is partitioned in an upper-lower direction. FIG. 12C shows a configuration example in which the internal space of the culture tank 10-2 having a rectangular box shape is partitioned in a left-right direction. FIG. 12D shows a configuration example in which the internal space of the culture tank 10-3 having a rectangular box shape is partitioned in the upper-lower direction. As shown in these configuration examples, the culture tank according to the present invention may be of any shape, and its internal space may be partitioned in any direction. The number of partitioned spaces may not necessarily be two. It may be partitioned into three or more spaces (see FIGS. 10A and B).

Furthermore, the configuration may be modified to a configuration in which partitioning plates 23-1, 23-2 and 23-3 as shown in FIGS. 12B, 12C and 12D can be moved to change the volume ratio of the first space R1 to the second space R2. In this case, the partitioning plates 23-1, 23-2 and 23-3 may, for example, be connected to a sliding mechanism or the like, which is driven by the controller 70 to change the volume ratio. Alternatively, a configuration in which the partitioning plates 23-1, 23-2 and 23-3 are operated like a diaphragm to change the volume ratio may also be applied.

### (Fourth Modification)

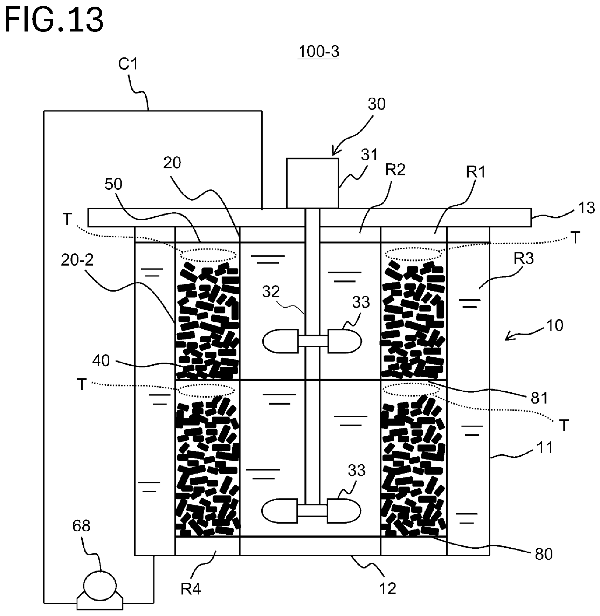

FIG. 13 is a diagram schematically illustrating the longitudinal cross-section of the cell culturing device 100-3 according to a fourth modification. As shown in FIG. 13, the cell culturing device 100-3 according to the fourth modification has a configuration in which the culture tank 10 is partitioned into three spaces concentrically using two cylindrical members 20 and 20-2 and further partitioned into two spaces in the upper-lower direction using a circular plate member 80. Note that the cylindrical member 20-2 (a second cylindrical member) has the same configuration as the cylindrical member 20 except for its diameter. The circular plate member 80 also has numerous holes or is formed into a mesh, and the numerous holes or the mesh allow the culture medium 50 to pass therethrough but prevent the scaffolds 40 from passing therethrough.

Therefore, the third space R3 formed on the outer periphery side of the culture tank 10 retains only the culture medium 50, similarly to the second space R2, such that no scaffolds 40 enter (or are present) therein. Similarly, a fourth space R4 formed on the lower portion of the culture tank 10 partitioned by the circular plate member 80 also retains only the culture medium 50 such that no scaffolds 40 enter therein.

The cell culturing device 100-3 is provided with the circulation flow passage C1 in which the culture medium 50 circulates among the third space R3, the second space R2 and the fourth space R4. The circulation flow passage C1 is provided with the circulation pump 68, and when the circulation pump 68 is driven, the culture medium 50 inside the third space R3 and the fourth space R4 is drawn in by the circulation pump 68 and discharged (returned) to the second space R2 via the circulation flow passage C1. The circulation flow passage C and the circulation pump 68 correspond to culture medium circulation means according to the present invention.

When the culture medium 50 inside the third space R3 and the fourth space R4 is drawn in by the circulation pump 68, the liquid pressures in the third space R3 and the fourth space R4 slightly drop, causing the liquid pressure in the second space R2 to be relatively higher. This causes the culture medium 50 to flow from the second space R2 to the third space R3 and the fourth space R4 via respective holes 21 of the cylindrical members 20 and 20-2, enabling the culture medium 50 to efficiently circulate. This further improves the uniformity of the supply of the culture medium 50 to the scaffolds 40, thereby further increasing cell culturing efficiency.

In the fourth modification, a shelf member 81 is provided at a position where it divides the culture tank 10 into two spaces with approximately equal volumes in the upper-lower direction. The shelf member 81, similarly to the circular plate member 80, has numerous holes or is formed into a mesh, allowing the culture medium 50 to pass therethrough but preventing the scaffolds 40 from passing therethrough. Therefore, a flow space T where the culture medium 50 can flow can be formed directly under the shelf member 81. The flow space T allows the culture medium 50 to pass therethrough, thereby further increasing culturing efficiency of the cells that adhere to the scaffolds 40.

Here, the shelf member 81 may be mounted to an inner circumferential surface of the cylindrical member 20-2 by any appropriate means. For example, a configuration in which the shelf member 81 is engaged with a protrusion, which is not shown in the figure, provided on the cylindrical member 20-2 allows it to be easily mounted and removed, thereby enabling the scaffolds 40 to be readily replaced.

Note that, although the fourth modification has a configuration in which the culture medium 50 is drawn in from the third space R3 and the fourth space R4 and returned to the second space R2, the configuration may be such that the culture medium 50 is drawn in from the second space R2 and returned to the third space R3 and the fourth space R4. The circular plate member 80 and the shelf member 81 may be configured using the same member or may be configured with holes or a mesh of different sizes.

### (Fifth Modification)

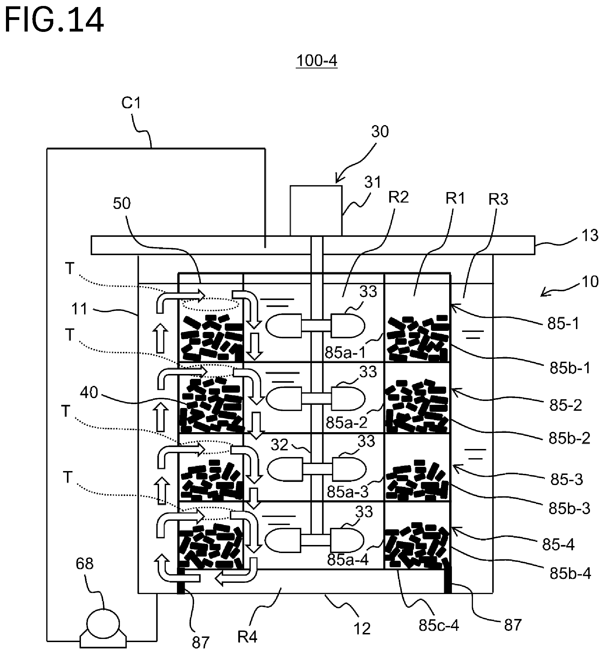

FIG. 14 is a diagram schematically illustrating the longitudinal cross-section of the cell culturing device 100-4 according to a fifth modification. As shown in FIG. 14, the cell culturing device 100-4 according to the fifth modification has four baskets 85-1, 85-2, 85-3 and 85-4 arranged coaxially in a stacked manner inside the culture tank 10 as partitioning means for partitioning its internal space. The basket 85-4 on the lowest tier is supported via a support member 87, thereby arranging the four baskets 85-1, 85-2, 85-3 and 85-4 at a distance equal to the height of the support member 87 from the bottom portion 12 of the culture tank 10. In other words, a space (the fourth space R4) is formed between the basket 85-4 on the lowest tier and the bottom portion 12 of the culture tank 10.

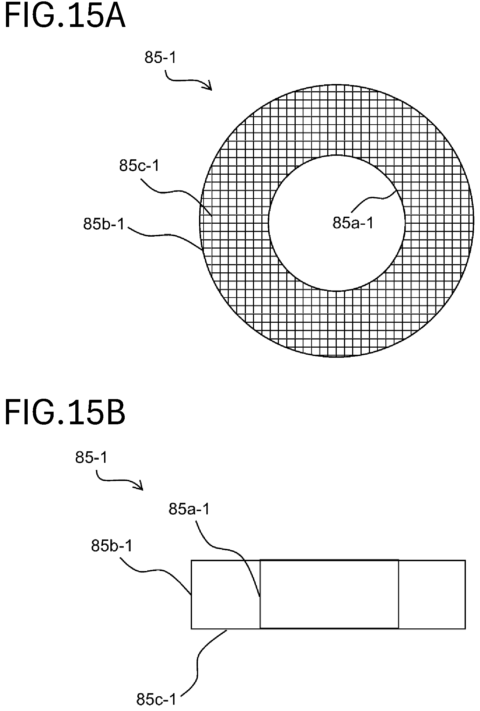

Next, details of a structure of baskets 85-1, 85-2, 85-3 and 85-4 are described. FIG. 15A is a plan view of the basket 85-1, and FIG. 15B is a longitudinal cross-sectional view of the basket 85-1. Note that since the four baskets 85-1, 85-2, 85-3 and 85-4 have the same configuration, the basket 85-1 is described here.

As shown in FIGS. 15A and 15B, the basket 85-1 includes an inner cylinder 85a-1, an outer cylinder 85b-1 and a bottom plate 85c-1 having an annular shape that connects the lower portions of the inner cylinder 85a-1 and the outer cylinder 85b-1. The inner cylinder 85a-1, the outer cylinder 85b-1 and the bottom plate 85c-1 each have numerous holes or are formed into a mesh, and the holes or the mesh allow the culture medium 50 to pass therethrough but prevent the scaffolds 40 from passing therethrough. This configuration allows the scaffolds 40 to be placed on the bottom plate 85c-1 and be accommodated in the space between the inner cylinder 85a-1 and the outer cylinder 85b-1.

When the baskets 85-1, 85-2, 85-3 and 85-4 are arranged in a stacked manner, as shown in FIG. 14, the second space R2 is formed inside the four inner cylinders 85a-1, 85a-2, 85a-3 and 85a-4, the first space R1 is formed between the four inner cylinders 85a-1, 85a-2, 85a-3 and 85a-4 and the four outer cylinders 85b-1, 85b-2, 85b-3 and 85b-4, the third space R3 is formed between the four outer cylinders 85b-1, 85b-2, 85b-3 and 85b-4 and the body portion 11 of the culture tank 10, and the fourth space R4 is formed between the bottom plate 85c-4 of the basket 85-4 on the lowest tier and the bottom portion 12 of the culture tank 10.

In addition, in the fifth modification, the stirring blades 33 are provided in a number of four, corresponding to the number of tiers of the baskets 85-1, 85-2, 85-3 and 85-4. This enables the culture medium 50 to efficiently circulate inside each of the baskets by means of each of the stirring blades 33. Needless to say, the number of stirring blades 33 may be fewer than the number of basket tiers.

The fifth modification enables the flow space T, where the culture medium 50 can flow, to be formed at the top portion of each of the baskets, while the scaffolds 40 are accommodated in the baskets 85-1, 85-2, 85-3 and 85-4. Therefore, the culture medium 50 circulates in the flow space T of each of the baskets as shown by white arrows in the figure. This enables the cells that adhere to the scaffolds 40 to be efficiently cultured.

Note that in the fifth modification, the shape of the basket may not necessarily be cylindrical. For example, any shape, such as a rectangular tubular basket with a bottom, may be adopted.

### (Sixth Modification)

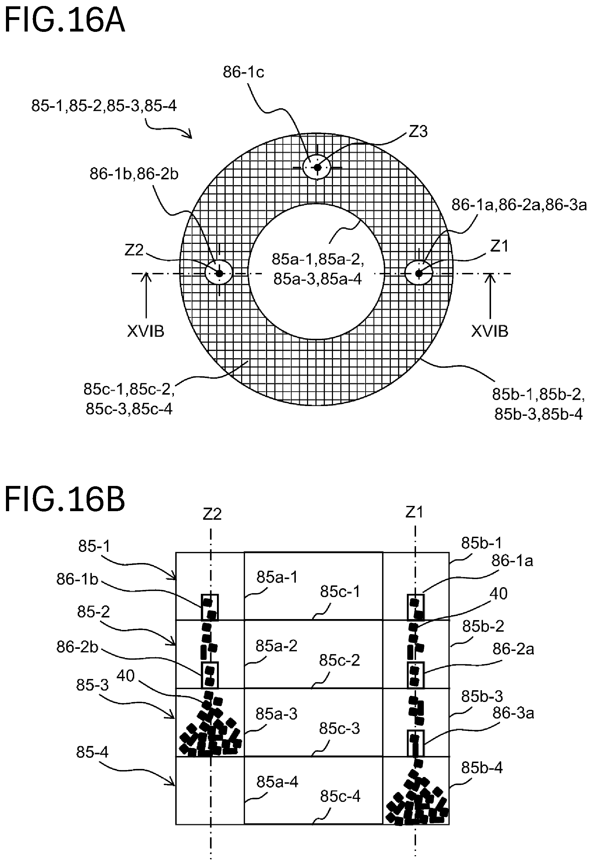

A sixth modification is based on the cell culturing device 100-4 according to the fifth modification with an improved structure of the basket. FIG. 16A is a plan view of the baskets 85-1, 85-2, 85-3 and 85-4 according to the sixth modification, and FIG. 16B is a longitudinal cross-sectional view of the baskets 85-1, 85-2, 85-3 and 85-4 according to the sixth modification arranged in a stacked manner, showing the cross-section along line XVIB-XVIB in FIG. 16A.

As shown in FIGS. 16A and 16B, the basket 85-1 on the highest tier includes three through passages 86-1a, 86-1b and 86-1c. In this example, the three through passages 86-1a, 86-1b and 86-1c may have the same shape or may have different shapes. The following description assumes that the three through passages 86-1a, 86-1b and 86-1c have the same shape.

The through passage 86-1a is formed by a cylindrical body that penetrates the bottom plate 85c-1 of the basket 85-1. The height (the length in the axial direction) of the through passage 86-1a is approximately 1/2 to 2/3 of the height of the basket 85-1. The through passage 86-1a is formed with a diameter that allows the scaffolds 40 to pass therethrough.

The basket 85-2 on a second tier from the top is provided with two through passages 86-2a and 86-2b, which have the same shape as the through passage 86-1a, and the through passage 86-2a and the through passage 86-2b are provided at a position where their centers are at the same location in plan view as the through passage on the highest tier 86-1a and as the through passage 86-1b, respectively. The basket 85-3 on a third tier from the top is provided with one through passage 86-3a having the same shape as the through passage 86-1a, and the through passage 86-3a is provided at a position where its center is at the same location in plan view as the through passage on the highest tier 86-1a and the through passage on the second tier 86-2a. Note that no through passage is provided in the basket 85-4 on the lowest tier.

With each of the baskets configured in this way arranged in a stacked manner, since the through passage 86-1a, the through passage 86-2a and the through passage 86-3a coincide in plan view (positioned along axis Z1) as shown in FIG. 16A, when the scaffolds 40 are supplied to the through passage 86-1a along axis Z1, the scaffolds 40 pass through the through passage 86-1a, the through passage 86-2a and the through passage 86-3a in that order, falling into and being accommodated inside the space between the inner cylinder 85a-4 and the outer cylinder 85b-4 of the basket 85-4 on the lowest tier as shown in FIG. 16B.

Similarly, since the through passage 86-1b and the through passage 86-2b coincide in plan view (positioned along axis Z2), when the scaffolds 40 are supplied to the through passage 86-1b along axis Z2, the scaffolds 40 pass through the through passage 86-1b and the through passage 86-2b in that order, falling into and being accommodated inside the space between the inner cylinder 85a-3 and the outer cylinder 85b-3 of the basket on the third tier 85-3 as shown in FIG. 16B.

In a case in which the scaffolds 40 are to be accommodated in the basket 85-2 on the second tier from the top, the scaffolds 40 may be dropped along axis Z3 into the through passage 86-1c.

According to the sixth modification, the scaffolds 40 can be easily supplied to the baskets 85-1, 85-2, 85-3 and 85-4, thereby increasing work efficiency compared to the fifth modification.

Note that, for example, the through passage 86-1a, the through passage 86-2a and the through passage 86-3a may not be arranged strictly along axis Z1, and all of the through passages may not necessarily be arranged coaxially, as long as they are arranged within a range that allows the scaffolds 40 to fall smoothly. Each of the through passages need to overlap at least partially in plan view. Arrangement of the through passage 86-1b and the through passage 86-2b is the same.

Needless to say, each of the first, second, third, fourth, fifth and sixth modifications described above can also be applied to the cell culturing device according to the second embodiment.

Here, the present invention can also be applied as a device for culturing cells other than animal-derived cells (for example, plant-derived cells or the like). When the culture tank 10 is small, a magnetic stirrer, for example, may be used as the stirring means 30. This offers the advantage of simplifying the structure.

The shape of the holes 21 provided in the cylindrical members 20, 20-1 and 20-2 is arbitrary. For example, they may be round holes, square holes or elongated holes. A cylindrical member provided with numerous slits may be applied to the present invention.

Although the embodiments described above employ a configuration in which the scaffolds 40 are collected and replenished using the level switches 65 and 66, weight sensors or the like may be used instead of the level switches 65 and 66. For example, weight of the culture tank 10 when the culture medium 50 is filled to the proper level PL and the scaffolds 40 are sufficiently supplied is set as a first threshold value, and the weight of approximately 50% of the first threshold value is set as a second threshold value. Then, a configuration in which the solenoid valve 60 closes when the weight sensor detects the second threshold value, which corresponds to S43 and S44 in FIG. 5, and the collection and replenishment process of the scaffolds 40 is terminated when the weight sensor detects the first threshold value, which corresponds to S46 in FIG. 5, enables control similar to that of the first embodiment.

Needless to say, the procedure shown in FIG. 4, for example, may be performed manually by the operator instead of the controller 70.

### List of Reference Signs

10: culture tank
11: body portion
12: bottom portion
13: lid body
15, 16: supply tube
20, 20-1: cylindrical member (partitioning means)
20-2: cylindrical member (second cylindrical member/partitioning means)
21: hole
23-1, 23-2, 23-3: partitioning plate (partitioning means)
25: expansion and contraction means
30: stirring means
31: motor
32: rotation shaft
33: stirring blade
40: scaffold
45: straightening plate
46: flow passage
50: culture medium
60: solenoid valve
65, 66: level switch
68: circulation pump (culture medium circulation means)
70: controller
80: circular plate member (partitioning means)
81: shelf member
85-1, 85-2, 85-3, 85-4: basket (partitioning means)
85a-1, 85a-2, 85a-3, 85a-4: inner cylinder
85b-1, 85b-2, 85b-3, 85b-4: outer cylinder
85c-1, 85c-2, 85c-3, 85c-4: bottom plate
86-1a, 86-1b, 86-1c: through passage
86-2a, 86-2b: through passage
86-3a: through passage
87: support member
100, 100-1, 100-2, 200: cell culturing device
C1: circulation flow passage (culture medium circulation means)
E1 to E4: electrical wiring
R1: first space
R1-1, R1-2, R1-3, R1-4: first divided space (first space)
R2: second space
R3: third space
R4: fourth space
T: flow space

## Claims

1. A cell culturing device for culturing cells, the cell culturing device comprising:
a culture tank for retaining a culture medium;
partitioning means for partitioning the culture tank into at least a first space and a second space; and
stirring means for stirring the culture medium,
wherein the first space is a space accommodating scaffolds to which the cells adhere,
the second space is a space in which the stirring means are provided, and
the partitioning means are configured to allow the culture medium to pass therethrough, and to disable entry of the scaffolds to the second space from the first space.

2. The cell culturing device according to claim 1,
wherein the volume ratio of the first space to the second space changes by operation of the partitioning means.

3. The cell culturing device according to claim 1,
wherein the partitioning means comprise a cylindrical member having numerous holes or being formed into a mesh,
a space having an annular shape between the culture tank and the cylindrical member forms the first space, and
the internal space of the cylindrical member forms the second space.

4. The cell culturing device according to claim 3,
wherein the cylindrical member is configured to be capable of decreasing and increasing its diameter inside the culture tank, and
decreasing or increasing the diameter of the cylindrical member changes the volume ratio of the first space to the second space.

5. The cell culturing device according to claim 1,
further comprising culture medium circulation means for drawing in the culture medium retained in the first space and returning it to the culture tank.

6. A cell culturing method using the cell culturing device according to claim 1, the cell culturing method comprising:
a step of supplying scaffolds to the first space and supplying the culture medium to the culture tank;
a step of driving the stirring means to stir the culture medium;
a step of collecting a portion of the scaffolds from the first space; and
a step of supplying new scaffolds into the first space and mixing them with the scaffolds remaining in the first space.

7. A cell culturing method using the cell culturing device according to claim 2, the cell culturing method comprising:
a step of supplying scaffolds to the first space and supplying the culture medium to the culture tank;
a step of driving the stirring means to stir the culture medium;
a step of collecting a portion of the scaffolds from the first space;
a step of increasing the volume of the first space by operation of the partitioning means;
a step of supplying new scaffolds to the first space whose volume has been expanded and mixing them with the scaffolds remaining in the first space; and
a step of returning the volume of the first space whose volume has been expanded to its original volume by the operation of the partitioning means.

8. The cell culturing device according to claim 3,
wherein the culture tank further comprises a third space having an annular shape that is a space outside the first space in a radial direction and is partitioned from the first space via a second cylindrical member having numerous holes or being formed into a mesh, and
the second cylindrical member is configured to allow the culture medium to pass therethrough, and to disable entry of the scaffolds to the third space from the first space.

9. The cell culturing device according to claim 8,
wherein the culture tank further comprises
a fourth space that is a space below the first space and the second space and partitioned from the first space and the second space via a circular plate member having numerous holes or being formed into a mesh, and
the circular plate member is configured to allow the culture medium to pass therethrough, and to disable entry of the scaffolds to the fourth space from the first space.

10. The cell culturing device according to claim 8 or 9,
further comprising at least one shelf member on which the scaffolds are placed,
wherein the at least one shelf member is arranged to partition the first space in an upper-lower direction, and
the at least one shelf member has numerous holes or is formed into a mesh, and allows the culture medium to pass therethrough but prevents the scaffolds from passing therethrough.

11. The cell culturing device according to claim 1,
wherein the culture tank is partitioned into three spaces concentrically arranged from the center in the order of the second space, the first space and the third space by the partitioning means,
further the cell culturing device includes a plurality of baskets that are the partitioning means,
each of the plurality of baskets comprises:
an inner cylinder having numerous holes or being formed into a mesh; an outer cylinder arranged concentrically with the inner cylinder and having numerous holes or being formed into a mesh; and a bottom plate having an annular shape and having numerous holes or being formed into a mesh that connects a bottom portion between the inner cylinder and the outer cylinder, and is configured to be capable of accommodating the scaffolds between the inner cylinder and the outer cylinder,
the plurality of baskets are arranged in a stacked manner along the central axis of the culture tank, and
for the culture tank, with the plurality of baskets arranged in a stacked manner, the first space and the third space are partitioned by the plurality of outer cylinders, and the first space and the second space are partitioned by the plurality of inner cylinders.

12. The cell culturing device according to claim 11,
wherein all of the plurality of baskets, except for the basket on the lowest tier, comprise at least one through passage having a cylindrical shape that penetrates respective bottom plates and through which the scaffolds can pass, and
the number of through passages increases from the lower-tier side to the upper-tier side of the plurality of baskets.

13. The cell culturing device according to claim 12,
wherein center positions of the through passages, when the plurality of baskets arranged in a stacked manner are viewed from an axial direction of the culture tank, coincide.

14. The cell culturing device according to claim 11,
wherein the basket positioned on the lowest tier from among the plurality of baskets is supported at a distance from the bottom portion of the culture tank.
